(19) Europäisches Patentamt
European Patent Office
Office européen des brevets

(11) EP 1 838 405 B1

(12) EUROPÄISCHE PATENTSCHRIFT

(45) Veröffentlichungstag und Bekanntmachung des Hinweises auf die Patenterteilung:
06.08.2008 Patentblatt 2008/32

(21) Anmeldenummer: 05821914.8

(22) Anmeldetag: 31.12.2005

(51) Int Cl.:
*B01D 3/42* (2006.01)      *C07D 307/89* (2006.01)
*C07C 51/573* (2006.01)

(86) Internationale Anmeldenummer:
PCT/EP2005/014159

(87) Internationale Veröffentlichungsnummer:
WO 2006/072463 (13.07.2006 Gazette 2006/28)

(54) **VERFAHREN ZUR HERSTELLUNG VON SPEZIFIKATIONSGERECHTEM PHTHALSÄUREANHYDRID**

METHOD FOR PRODUCING PHTHALIC ANHYDRIDE THAT CONFORMS TO SPECIFICATIONS

PROCEDE DE PRODUCTION D'ANHYDRIDE PHTALIQUE CONFORME AUX SPECIFICATIONS

(84) Benannte Vertragsstaaten:
AT BE BG CH CY CZ DE DK EE ES FI FR GB GR HU IE IS IT LI LT LU LV MC NL PL PT RO SE SI SK TR

(30) Priorität: 07.01.2005 DE 102005000957

(43) Veröffentlichungstag der Anmeldung:
03.10.2007 Patentblatt 2007/40

(73) Patentinhaber: BASF SE
67056 Ludwigshafen (DE)

(72) Erfinder:
• PESCHEL, Werner
67251 Freinsheim (DE)
• KUMMER, Matthias
67273 Weisenheim (DE)
• BECHTEL, Marcus
69221 Dossenheim (DE)

(56) Entgegenhaltungen:
EP-A- 1 233 012          WO-A-01/14308
US-A- 3 699 008

**Beschreibung**

Die vorliegende Erfindung betrifft ein Verfahren zur Herstellung von spezifikationsgerechtem Phthalsäureanhydrid durch die destillative Reinigung von rohem Phthalsäureanhydrid, wobei man das rohe Phthalsäureanhydrid der Destillationskolonne oberhalb eines Seitenabzugs zuführt, die Leichtsieder am Kopf der Kolonne oder in der Nähe des Kopfes der Kolonne abzieht und das spezifikationsgerechte Phthalsäureanhydrid aus dem Seitenabzug der Kolonne entnimmt.

Phthalsäureanhydrid (im folgenden auch "PSA" genannt) ist eine bedeutsame Grundchemikalie der chemischen Industrie. Es dient zu einem beträchtlichen Teil als Ausgangsstoff für Dialkylphthalate, die in großen Mengen als Weichmacher für Kunststoffe wie PVC zum Einsatz kommen. Rohes PSA wird in der Technik aus Naphthalin und/oder o-Xylol durch katalytische Oxidation in der Gasphase hergestellt. Vorzugsweise setzt man für die oben genannten Zwecke ein PSA ein, das aus o-Xylol hergestellt wurde. Die Austräge der genannten gebräuchlichen Herstellverfahren weisen, bezogen auf ihr Gesamtgewicht, üblicherweise mehr als 99 Gew.-% PSA auf. Dieses rohe PSA wird meist in flüssiger Form oder als Feststoff anhand von Abscheidern isoliert.

In Abhängigkeit vom gewählten Herstellverfahren und dabei besonders von den Ausgangsstoffen und den Katalysatoren enthält das Produkt ein jeweils spezifisches Spektrum von Verunreinigungen und Nebenprodukten (vgl. z.B.: H. Suter: "Phthalsäureanhydrid und seine Verwendung", Dr. Dietrich Steinkopff Verlag, Darmstadt, 1972, Seite 39 ff.; im Folgenden kurz "Suter" genannt).

Am Markt wird eine PSA-Qualität mit folgenden Spezifikationsgrenzen erwartet:

| | |
|---|---|
| Erstarrungspunkt (°C) | min. 130,8 |
| Massenanteile (Gew.-%): | |
| PSA | min. 99,8 |
| MSA | max. 0,05 |
| Benzoesäure | max. 0,10 bzw. |
| | max. 0,002 bei Riechstoffqualität |
| Phthalsäure | max. 0,1 |
| Schmelzfarbzahl (Hazen) | max. 20 |
| Hitzefarbzahl (Hazen) | max. 40 |

In der Technik hat sich über die lange Zeit, in der PSA bereits im industriellen Maßstab hergestellt wird, die Abtrennung dieser Nebenprodukte mittels Destillation durchgesetzt (vgl. z.B.: "Ullmann's Encyclopedia of Industrial Chemistry", 5. Edition, Vol. A20, VCH Verlagsgesellschaft mbH, Weinheim, 1992, Seiten 181 bis 189; im Folgenden kurz "Ullmann" genannt; Kirk-Othmer "Encyclopedia of Chemical Technology", 4. Edition, Vol. 18, John Wiley & Sons, New York, 1996, Seiten 997 bis 1006, im Folgenden kurz "Kirk-Othmer" genannt). Niedrig siedende und/oder azeotrop destillierende Verunreinigungen, teilweise mit intensiver Eigenfarbe, bereiten dem Fachmann trotz ihrer vergleichsweise geringen Anteile dabei jedoch große Mühe.

Die Destillation - vor allem ihre unter wirtschaftlichen Aspekten häufig besonders interessante, kontinuierliche Durchführung - erfolgt üblicherweise mittels zweier Kolonnen, um ein hinreichend reines PSA zu erhalten. Im ersten Schritt werden dabei in der Regel die Leichtsieder (beispielsweise die Hauptmengen an Benzoesäure, Maleinsäureanhydrid, Citraconsäureanhydrid), also Stoffe mit einem Siedepunkt unterhalb des Siedepunktes des PSAs abgetrennt; in einem zweiten Schritt destilliert man dann PSA von den Schwersiedern (beispielsweise Phthalsäure, bestimmte farbgebende Komponenten, Kondensationsprodukte aus Inhaltsstoffen des rohen PSAs), also Stoffen mit höheren Siedepunkten als dem von PSA bzw. von undestillierbaren Bestandteilen, ab.

Andere Verfahren zur Reinigung von rohem Phthalsäureanhydrid umfassen dessen thermische und gegebenenfalls zusätzliche chemische Behandlung vor dessen Destillation, beispielsweise das Verfahren gemäß US-A 4,547,578, oder die Absorption von gasförmigem PSA in Paraffinöl, Auskristallisation des PSA und anschließende destillative Reinigung der wieder aufgeschmolzenen PSA-Kristalle gemäß US-A 4,008,255. Alle diese Verfahren verlangen einen hohen apparativen Aufwand, haben einen hohen Energieverbrauch und sind folglich unwirtschaftlich.

In "Suter" (dort auf Seite 45) wird bereits auf eine einstufige kontinuierliche Destillation von PSA verwiesen (Ruhröl, Europa-Chemie Heft 21, S. 7 (1965)), wobei keine Einzelheiten genannt werden.

Besonders hohe Anforderungen werden an solche aus PSA synthetisierten Ester der Phthalsäure gestellt, welche als Lösungs- oder Streckmittel in Parfümen oder Kosmetika verwendet werden sollen. Die Anwesenheit von geringen Mengen Maleinsäure, Citraconsäure sowie deren Anhydriden und vor allem von Benzoesäure im PSA führt jedoch zu Veresterungsprodukten dieser Stoffe, welche intensive, charakteristische Geruchsnoten aufweisen, etwa eine diffus-fruchtige Note im Falle von Benzoesäureethylester. Derartige Verunreinigungen sind üblicherweise im Anschluss an die Ester-Synthese mittels eines kombinierten Wasch- und Extraktionsschrittes zu entfernen.

Dieses Verfahren ist sehr aufwendig und macht normalerweise die vorangehende, übliche Destillation des rohen PSAs nicht entbehrlich.

Die Aufgabe, die im rohem PSA nur in geringen Mengen vorliegenden, je nach Verwendungszweck des PSA sich jedoch sehr störend auswirkenden Verunreinigungen, entsprechend den vom Kunden geforderten Spezifikationen abzutrennen, wird gemäß WO 01/14308 mittels eines einstufigen destillativen Verfahrens zu lösen versucht. Dort wird spezifikationsgerechtes PSA durch destillative Reinigung von rohem PSA gewonnen, indem man rohes PSA einer Destillationskolonne, die bei vermindertem Druck betrieben wird, zuführt, die Leichtsieder am Kopf oder in der Nähe des Kopfes der Destillationskolonne und das spezifikationsgerechte PSA über einen Seitenabzug aus der Kolonne entnimmt.

Bei einer theoretischen Trennstufenzahl von insgesamt ungefähr 18 wird gemäß Beispiel von WO 01/14308 bei einem Rücklaufverhältnis von 0,6 ein Phthalsäureanhydrid mit einem Benzoesäuregehalt von 30 Gew.-ppm erhalten. Insbesondere für Riechstoffanwendungen wird eine weitere destillative Absenkung des Benzoesäuregehaltes im PSA gewünscht. Darüber hinaus wird bei diesem Verfahren infolge des hohen Rücklaufverhältnisses viel Energie verbraucht.

Die weitergehende destillative Verringerung des Benzoesäuregehaltes gestaltet sich jedoch schwierig. Zwar erscheint es möglich, durch eine Verringerung des Rücklaufverhältnisses, mehr Benzoesäure über den Kopf der Kolonne abzuziehen, diese Maßnahme führt jedoch zu einem erhöhten Austrag von PSA über den Kopf der Kolonne zusammen mit den Leichtsiedern und folglich zu erheblichen PSA-Verlusten. Dieser Effekt ist aus EP-A 1 233 012 ersichtlich, das ebenfalls ein einstufiges Verfahren zur Destillation von PSA zum Gegenstand hat. Im einzigen Beispiel von EP-A 1 233 012 wird bei einem Zulauf zur Kolonne von 1000 g/h Phthalsäureanhydrid und einem Rücklauf von 530 g/h, entsprechend einem Rücklaufverhältnis von 0,53, zwar ein Phthalsäureanhydrid erhalten, dessen Benzoesäuregehalt nur noch 15 Gew.-ppm beträgt, die Wiedergewinnungsrate für das Phthalsäureanhydrid sinkt dabei jedoch auf nur 97%, wodurch das Verfahren unwirtschaftlich wird.

Der vorliegenden Erfindung lag deshalb die Aufgabe zugrunde, ein gegenüber dem Stand der Technik verbessertes Verfahren zur destillativen Erzeugung spezifikationsgerechten PSA zur Verfügung zu stellen. Insbesondere sollte das Verfahren in der Lage sein, bei niedrigem Energieverbrauch und geringen PSA-Verlusten spezifikationsgerechtes PSA mit einem niedrigen Gehalt an Benzoesäure und anderen Leichtsiedern ohne eine Beeinträchtigung der Farbzahl des PSA zu liefern.

Dementsprechend wurde ein Verfahren zur Herstellung von spezifikationsgerechtem Phthalsäureanhydrid durch die destillative Reinigung von rohem Phthalsäureanhydrid bei vermindertem Druck, wobei man das rohe Phthalsäureanhydrid der Destillationskolonne oberhalb eines Seitenabzugs zuführt, die Leichtsieder am Kopf der Kolonne oder in der Nähe des Kopfes der Kolonne abzieht und das spezifikationsgerechte Phthalsäureanhydrid aus dem Seitenabzug der Kolonne entnimmt, gefunden, das dadurch gekennzeichnet ist, dass man eine Destillationskolonne verwendet, deren Anzahl an oberhalb der Zuleitung des rohen Phthalsäureanhydrids in die Destillationskolonne befindlichen theoretischen Trennstufen 10 bis 20 beträgt und die Kolonne bei einem Rücklaufverhältnis von 0,1 bis 0,5 betreibt.

Erfindungsgemäß wird die im erfindungsgemäßen Verfahren verwendete Destillationskolonne bei einem Rücklaufverhältnis von 0,1 bis 0,5, vorzugsweise von 0,2 bis 0,45 und besonders bevorzugt von 0,25 bis 0,45 betrieben. Als Rücklaufverhältnis ("RLV") wird der Quotient

$$RLV = \frac{R\ddot{u}cklaufmenge/Zeiteinheit}{Zulauf\ zur\ Kolonne/Zeiteinheit} \qquad (1)$$

bezeichnet.

Die im erfindungsgemäßen einzusetzende Destillationskolonne wird erfindungsgemäß so ausgelegt, dass die Anzahl der oberhalb der Zuleitung des rohen PSA in die Kolonne befindlichen theoretischen Trennstufen 10 bis 20, vorzugsweise 10 bis 15, beträgt.

Als theoretische Trennstufe, in der Literatur oftmals auch als "theoretischer Boden" bezeichnet, wird die Kolonneneinheit definiert, die eine Anreicherung an leichter flüchtiger Komponente entsprechend dem thermodynamischen Gleichgewicht zwischen Flüssigkeit und Dampf in einem einzigen Destillationsvorgang gemäß Gleichung (2)

$$\frac{Y_1}{1-Y_1} = \alpha \frac{X_1}{1-X_1} \qquad (2)$$

bewirkt. In dieser Gleichung stellt $X_1$ den Molenbruch der leichter siedenden Komponente in der flüssigen Phase, $Y_1$ den Molenbruch der leichter siedenden Komponente im Dampfraum und die Konstante $\alpha$ die relative Flüchtigkeit dar, die sich aus dem Quotienten des Dampfdrucks P der reinen Komponenten A und B des zu destillierenden Gemisches

$$\frac{P_A}{P_B} = \alpha \qquad (3)$$

ergibt.

Ist der Unterschied von $P_A$ und $P_B$ gering, kann folglich eine vollständige Trennung der Komponenten in einem einzigen Destillationsvorgang, also in einer theoretischen Trennstufe, nicht erzielt werden. Um eine vollständige Trennung der beiden Komponenten zu erzielen muss der einzelne Destillationsvorgang - in der Regel vielmals - wiederholt werden, wobei man dann im Falle einer Zusammenfassung dieser vielen einzelnen Destillationsvorgänge in einer Kolonne von einer Rektifikation spricht. Die in einer solchen Destillationskolonne installierten "praktischen" Böden, die in unterschiedlicher Form ausgestaltet sein können, stellen gewissermaßen jeder für sich eine neue Destillationsblase dar. Im Allgemeinen erreichen diese "praktischen" Böden die Wirkung einer theoretischen Trennstufe (also eines theoretischen Bodens) nicht. Dementsprechend wird die Trennleistung einer Destillationskolonne üblicherweise durch die Anzahl n der darin vorhandenen theoretischen Trennstufen angegeben. Die Anzahl n der in einer Kolonne oder bei Bezugnahme auf einen Teil der Kolonne in diesem Teil der Kolonne vorhandenen theoretischen Trennstufen kann aus Gleichung (4)

$$\frac{Y_n}{1-Y_n} = \alpha^n \frac{X_1}{1-X_1} \qquad (4)$$

worin $Y_n$ den Molenbruch der leichter siedenden Komponente im Dampfraum nach n-maliger Wiederholung des Verdampfungs-Kondensations-Vorgangs darstellt, durch deren Auflösung nach n berechnet werden.

Es ist offensichtlich, dass sich die vorstehenden Erläuterungen zur Definition einer theoretische Trennstufe in dieser Form nur für ein sich ideal oder annähernd ideal verhaltendes Zwei-Komponenten-System gelten. Obgleich das erfindungsgemäß zu reinigende PSA eine Vielzahl unterschiedlicher Verunreinigungen enthält, bezieht sich die erfindungsgemäße Angabe der Anzahl der oberhalb des Roh-PSA-Zulaufs in die Kolonne befindlichen theoretischen Trennstufen allein auf das System der beiden zu trennenden Komponenten Benzoesäure und Phthalsäureanhydrid.

Die vorstehenden Ausführungen zur Bedeutung des Begriffs der theoretischen Trennstufe dienen lediglich der Erläuterung und Klarstellung dieses Begriffes im Rahmen der vorliegenden Erfindung und sind dem Fachmann z.B. aus Lehrbüchern, wie Organikum (14. Aufl., S. 42 - 44 und S. 50 - 60, VEB Deutscher Verlag der Wissenschaften, Berlin 1975) oder Vauck; Müller, Grundoperationen chemischer Verfahrenstechnik (11. Aufl.; Kapitel 10.4.2 Gegenstromdestillation, S. 710 - 761; Deutscher Verlag für Grundstoffindustrie, Stuttgart 2000), oder aus Zusammenstellungen, wie in Kirk-Othmer, Encyclopedia of Chemical Technology (4th Ed., Vol. 8, Kapitel: Distillation, S. 311 - 358, John Wiley & Sons, New York 1993), die auch noch detailliertere und weitergehendere Informationen zur Durchführung von Destillationen enthalten, geläufig.

Die Gesamtzahl der theoretischen Trennstufen der im erfindungsgemäßen Verfahren einsetzbaren Destillationskolonnen beträgt im Allgemeinen 15 bis 40, vorzugsweise 20 bis 30 und besonders bevorzugt 22 bis 26 theoretische Trennstufen.

Während die Einhaltung der erfindungsgemäßen Anzahl der oberhalb des Zulaufs des Roh-PSA in die Kolonne befindlichen Trennstufen für die Lösung der der Erfindung zugrundeliegenden Aufgabe kritisch ist, bestehen für die Auslegung der Anzahl an theoretischen Trennstufen in den beiden unterhalb des Zulaufs des Roh-PSA gelegenen Abschnitten der Kolonne; nämlich dem Abschnitt zwischen Zulauf des Roh-PSA in die Kolonne und dem Seitenabzug und dem Abschnitt der Kolonne unterhalb des Seitenabzugs gewisse Variationsmöglichkeiten. Im Allgemeinen wird der Abschnitt unterhalb des Zulaufs des Roh-PSA bis zum Seitenabzug so ausgelegt, dass er eine Anzahl theoretischer Trennstufen von in der Regel 3 bis 15, vorzugsweise von 6 bis 12 hat. Der Abschnitt der Destillationskolonne unterhalb des Seitenabzugs wird im Allgemeinen so ausgelegt, dass er eine Anzahl theoretischer Trennstufen von in der Regel 2 bis 8, vorzugsweise von 3 bis 6 hat.

Zur Durchführung des erfindungsgemäßen Verfahrens können an sich übliche Destillationskolonnen eingesetzt werden, beispielsweise Bodenkolonnen, Füllkörperkolonnen, Packungskolonnen oder Kolonnen, in denen eine Kombination der Merkmale der vorstehend genannten Kolonnentypen vorliegt. Entsprechend dem verwendeten Kolonnentyp kann dieser mit an sich üblichen Einbauten wie Böden, Füllkörpern oder Packungen ausgestattet sein, beispielsweise mit Glockenböden, Tunnelböden, Ventilböden, Siebböden, Dual-Flow-Böden und/oder Gitterböden, mit Pall-Ringen®, Berl®-Sattelkörpern, Netzdrahtringen, Raschig-Ringen®, Intalox®-Sätteln, Interpak®-Füllkörpern und Intos® aber auch mit geordneten Packungen, wie beispielsweise Sulzer-Mellapak®, Sulzer-Optiflow®, Kühni-Rombopak® und Montz-Pak®sowie Gewebepackungen. Im Bereich unterhalb des Zulaufes der Kolonne werden vorzugsweise Einbauten gewählt, die auch feststofftauglich sind, insbesondere Dual-Flow-Böden. Geeignet sind hierfür in der Regel Böden und Füllkörper der oben genannten Bauarten.

Entsprechend der Trennleistung der verwendeten Kolonneneinbauten errechnet sich die Anzahl der zur Erzielung der erfindungsgemäß einzustellenden Anzahl theoretischen Trennstufen oberhalb des Roh-PSA-Zulaufs in die Destillationskolonne bzw. die zur Einstellung der gewünschten Anzahl an theoretischen Trennstufen in den beiden unterhalb des Zulaufs des Roh-PSA in die Destillationskolonne gelegenen Abschnitten der Kolonne benötigte Anzahl an praktischen Böden und demzufolge auch die Höhe der Kolonne. Hingegen dient als Basis zur Auslegung des Durchmessers der Destillationskolonne der mit der Kolonne angestrebte Durchsatz oder mit anderen Worten, deren gewünschte Produktionskapazität. In Kenntnis der erfindungsgemäßen Auslegungskriterien für die erfindungsgemäß einzusetzende Destillationskolonne können die dazu nötigen Berechnungen nach dem Fachmann geläufigen, ingenieurtechnischen Berechnungsmethoden durchgeführt werden.

Zur Erzeugung des Rücklaufs auf die Destillationskolonne wird die am Kopf oder in der Nähe des Kopfes der Kolonne gasförmig abgezogene Leichtsiederfraktion in einem kondensator kondensiert und das Kondensat entsprechend dem gewünschten Rücklaufverhältnis wieder in den Kopf der Kolonne oder in der Nähe des Kolonnenkopfes in die Kolonne zurückgeführt.

Die Destillationskolonne kann mit handelsüblichen Verdampfern betrieben werden. Zweckmäßigerweise können Sumpfverdampfer verwendet werden, wobei diese vorteilhaft als Fallfilmverdampfer ausgestaltet werden. Die Verwendung von Fallfilmverdampfern ermöglicht eine aufgrund der geringen mittleren Verweilzeit der Sumpfflüssigkeit im Verdampfer schonende Verdampfung der Sumpfflüssigkeit, wodurch die Neigung zur Feststoffbildung als auch zur Bildung von Zersetzungsprodukten während der Verdampfung vermindert und somit die Rein-PSA Ausbeute als auch die Wirtschaftlichkeit des Verfahrens verbessert wird.

Die Schwersieder können kontinuierlich oder diskontinuierlich aus dem Sumpf der Destillationskolonne oder dem flüssigen Verdampfungsrückstand im Verdampfer entnommen und entsorgt werden.

Die Kolonne kann im Allgemeinen bei einem absoluten Druck am Kolonnenkopf von 0,05 bis 0,5, bevorzugt 0,1 bis 0,3, besonders bevorzugt bei 0,12 bis 0,20 bar betrieben werden.

Die Temperaturen in der Kolonne liegen am Kolonnenkopf im Allgemeinen bei 160 bis 220, vorzugsweise bei 170 bis 200 und vor allem bei 175 bis 185°C und am Kolonnensumpf bei 220 bis 260, vorzugsweise bei 225 bis 250 und vor allem bei 230 bis 245°C.

Die Temperatur am Seitenabzug liegt im Allgemeinen bei 210 bis 250 und vorzugsweise bei 220 bis 240°C.

Die Destillation kann diskontinuierlich oder vorzugsweise kontinuierlich durchgeführt werden. Das rohe PSA kann der Kolonne in gasförmiger oder bevorzugt in flüssiger Form über den Zulauf zugeführt werden. Das gereinigte, spezifikationsgerechte PSA kann dem Seitenabzug der Destillationskolonne, der sich unterhalb des Zulaufs des Roh-PSA in die Destillationskolonne befindet, gasförmig entnommen werden.

In einer bevorzugten Ausführungsform des erfindungsgemäßen Verfahrens können bei Verwendung von Bodenkolonnen Tropfenabscheider am Seitenabzug für das gasförmige Rein-PSA innerhalb oder außerhalb der Destillationskolonne installiert werden.

Mit dem erfindungsgemäßen Verfahren ist es möglich, ein PSA mit einem Gehalt an Benzoesäure von weniger als 20 ppm, vorzugsweise zwischen 5 und weniger als 20 ppm zu gewinnen. Das erfindungsgemäße Verfahren eignet sich ganz besonders für ein rohes PSA, welches einen Gehalt an PSA von 95,0 bis 99,8 Gew.-% und einen Gehalt an Benzoesäure von 0,1 bis 5,0 und vor allem von 0,2 bis 1,0 Gew.-% aufweist.

Besonders eignet sich das erfindungsgemäße Verfahren für rohes PSA, wie es durch katalytische Gasphasenoxidation von o-Xylol erhalten wird mit einem Gehalt von vorzugsweise mehr als 95 und insbesondere mehr als 98 Gew.-% PSA.

Mit dem erfindungsgemäßen Verfahren wird eine Schmelzfarbzahl des PSA von weniger als 10 APHA und eine Hitzefarbzahl von weniger als 20 APHA erreicht.

Das spezifikationsgerechte PSA wird üblicherweise unmittelbar nach der Entnahme aus der Kolonne gekühlt und als Flüssigkeit oder, nach dem Erstarren, als Festkörper erhalten. Ein noch höherer Reinheitsgrad kann gewünschtenfalls erreicht werden, indem man das PSA beispielsweise über eine Seitenkolonne feindestilliert oder ein Trennblech achsial über einen bestimmten Bereich in der Kolonne (sog. Petlyuk-Verschaltung) anbringt. Auch ein Umkristallisieren kommt hier in Betracht.

Die Wiedergewinnungsrate an PSA am Seitenabzug bezogen auf den Gehalt an PSA im Zulauf zur Kolonne liegt in der Regel bei 98 % und höher.

Die Reinheit des so erhaltenen PSAs lässt sich anhand allgemein bekannter analytischer Methoden wie der Gaschromatographie, der UV-Spektroskopie und der Säure-Base-Titration ermitteln. Weil für die meisten Verwendungszwecke ein PSA ohne verfärbende Verunreinigungen benötigt wird, kommt der Charakterisierung durch die sogenannten Farbzahlen - vorrangig der Schmelzfarbzahl und der Hitzefarbzahl - besondere Bedeutung zu. Farbliche Veränderungen des PSAs unter thermischer Belastung sind deswegen von praktischer Bedeutung, weil PSA normalerweise im geschmolzenen Zustand - etwa bei 160°C - gelagert und transportiert wird. Insbesondere die Schmelzfarbzahl (APHA/ Hazen-Farbskala, vgl. W. Liekmeier, D. Thybusch: Charakterisierung der Farbe von klaren Flüssigkeiten, Editor: Bodenseewerk Perkin-Elmer GmbH, Überlingen, 1991) wird im Allgemeinen so ermittelt, dass man die Farbzahl von PSA unmittelbar nach der Probenahme bei einer Temperatur von 160°C bestimmt. Weiterhin wird die Hitzefarbzahl im Allgemeinen so ermittelt, dass man das PSA 90 Minuten lang bei 250°C hält und dann die Farbzahl misst.

Durch die Anwendung der erfindungsgemäßen Maßnahmen wird die der Erfindung zugrundeliegende Aufgabe sehr gut gelöst. Aufgrund des geringen Rücklaufverhältnisses gelingt die Abtrennung der Benzoesäure und anderer im Roh-PSA enthaltener Leichtsieder bis auf sehr geringe Restgehalte bei gegenüber dem Stand der Technik deutlich verringertem Energieverbrauch. Infolge der Erhöhung der Anzahl der theoretischen Trennstufen oberhalb der Zuleitung des Roh-PSA in die Destillationskolonne werden andererseits die Verluste an PSA durch Austrag mit den Leichtsiedern minimiert. Entgegen den Erwartungen führen diese Maßnahmen auch nicht zu einer Erhöhung der Farbzahl im über den Seitenabzug der Destillationskolonne ausgetragenen Rein-PSA. Dies ist deshalb überraschend als der am Seitenabzug vorbei in den Sumpf der Kolonne ablaufende Rücklaufstrom infolge des geringen angewandten Rücklaufverhältnisses hochkonzentriert an farbgebenden Verunreinigungen ist, weshalb nachteilige Auswirkungen auf die Farbzahl des Rein-PSA zu erwarten waren. Die Einhaltung einer niedrigen Farbzahl im gereinigten PSA ist neben einem geringen Gehalt an Leichtsiedern, insbesondere Benzoesäure, besonders kritisch für die spätere Verwendung des PSA, da die farbgebenden Verunreinigungen mit der Zeit, insbesondere bei thermischer Belastung - das PSA wird üblicherweise in geschmolzenem Zustand transportiert und gelagert - und/oder in Gegenwart von Sauerstoff nachdunkeln und zur Vergilbung oder braunen Verfärbung der damit hergestellten Produkte führen, wodurch diese praktisch unverkäuflich werden.

Die Erfindung soll anhand der folgenden Beispiele erläutert werden.

Beispiele

A) Verwendete Apparatur

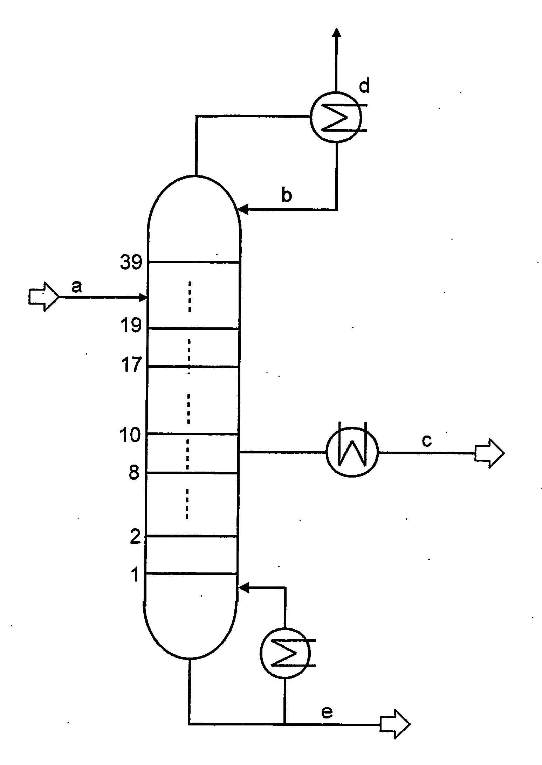

Es wurde eine Bodenkolonne gemäß der schematischen Zeichnung 1 verwendet. Die Anzahl der Böden in der Kolonne konnte variiert werden. Für die Durchführung der Versuche wurden entsprechend den Beispielen 32 bis 39 Ventilböden installiert, entsprechend einer Anzahl von 22 bis 27 theoretischen Trennstufen. Die Kolonne hatte einen Durchmesser von 50 mm. Der Seitenabzug (c) befand sich zwischen dem 10. und 11. Boden über dem Sumpf (überschlägig im Bereich zwischen der siebten und achten theoretischen Trennstufe), der Zulauf des Roh-PSA (a) befand sich zwischen dem 21. und 22. Boden über dem Sumpf (überschlägig im Bereich der vierzehnten theoretischen Trennstufe). In der Zeichnung sind der 1. und 2. Boden eingezeichnet, die übrigen Böden sind durch eine senkrechte, unterbrochene Linie angedeutet.

B) Eingesetztes rohes PSA

Es kam ein rohes PSA zur Destillation, das durch Gasphasenoxidation von o-Xylol an einem Festbett in Gegenwart eines Katalysators, bestehend aus einem Trägerkern und den darauf schalenförmig aufgebrachten katalytisch wirksamen Metalloxiden Cäsiumoxid (berechnet als 0,4 Gew.-% Cäsium), Vanadiumoxid (4 Gew.-%) und Titandioxid (95,6 Gew.-%), hergestellt worden war (vgl. die WO-A 01/14308). Die Beladung im Reaktor betrug 86 g o-Xylol pro Nm$^3$ Luft. Die Reaktortemperatur lag zwischen 350 und 450°C.

Das so erhaltene rohe PSA hatte folgende gewichtsbezogene Zusammensetzung:

| | |
|---|---|
| 99,24 Gew.-% | PSA |
| 0,2 Gew.-% | Benzoesäure |
| 200 ppm | Maleinsäureanhydrid |
| 20 ppm | Citraconsäureanhydrid |
| 0,5 Gew.-% | Phthalsäure |

und der Rest zu 100 Gew.-% sonstige Stoffe.

C) Allgemeingültige Verfahrensschritte

Die Schmelzfarbzahl wurde unmittelbar nach der Entnahme der Probe aus dem destillierten PSA ermittelt. Die Hitzefarbzahl wurde wie folgt ermittelt: Eine Probe PSA wurde in einem Wärmeschrank 1,5 Stunden bei einer Temperatur von 250°C getempert. Anschließend wurde die Farbzahl gemessen.

Beispiel 1

Vergleichsbeispiel: Destillation gemäß dem Stand der Technik (EP-A 1 233 012); Bezugszeichen verweisen auf die Abbildung

1000 g des rohen PSAs gemäß voranstehendem Abschnitt B) wurden der Kolonne stündlich kontinuierlich zugeführt (a). In diesem Zeitraum wurden der Kolonne eine Energiemenge von 720 kJ/kg rohen PSA zugeführt. Bei einem Rücklauf von 530 g (b), einem absoluten Druck von 0,17 bar am Kolonnenkopf, einer Temperatur von 198°C am Kolonnenkopf und 238°C am Kolonnensumpf wurden in der gleichen Zeit 970 g gereinigtes PSA über den Seitenabzug bei 221°C entnommen, kondensiert und isoliert (c). Das Rücklaufverhältnis in der Kolonne betrug dementsprechend 0,53 und die Ausbeute an derart gereinigtem PSA betrug 97,8 Gew.-%, bezogen auf das der Kolonne zugeführte rohe PSA. Der Kopfabzug über (d) wurde in eine Kühlfalle kondensiert und betrug ca. 7 g; der Sumpfabzug über (e) betrug ca. 15 g und enthielt die Schwersieder und nichtdestillierbare Anteile. Die Analyse des über den Seitenabzug bei (c) isolierten PSAs ergab folgende gewichtsbezogene Zusammensetzung:

| 99,97 Gew.-% | PSA |
| 15 ppm | Benzoesäure |
| < 10 ppm | Maleinsäureanhydrid |
| < 10 ppm | Citraconsäureanhydrid |
| 0,02 Gew.-% | Phthalsäure |

und der Rest zu 100 Gew.-% sonstige Stoffe.
Die Schmelzfarbzahl betrug 5-10 APHA. Die Hitzefarbzahl des PSA betrug 10 - 20 APHA.

Beispiel 2

Erstes erfindungsgemäßes Beispiel

1150 g des rohen PSAs gemäß dem vorangehenden Abschnitt B) wurden der Kolonne stündlich kontinuierlich zugeführt (a). In diesem Zeitraum wurden der Kolonne eine Energiemenge von 690 kJ/kg zugeführt. Bei einem Rücklauf von 510 g (b), einem absoluten Druck von 150 mbar am Kolonnenkopf, einer Temperatur von 192°C am Kolonnenkopf und 235°C am Kolonnensumpf wurden in der gleichen Zeit 1130 g gereinigtes PSA über den Seitenabzug bei 224°C entnommen, kondensiert und isoliert (c). Das Rücklaufverhältnis in der Kolonne betrug dementsprechend 0,44 und die Ausbeute an derart gereinigtem PSA betrug 99,0 Gew.-%, bezogen auf das der Kolonne zugeführte rohe PSA. Der Kopfabzug über (d) wurde in eine Kühlfalle kondensiert und betrug ca. 5g; der Sumpfabzug über (e) betrug ca. 15 g und enthielt die Schwersieder und nichtdestillierbare Anteile. Die Analyse des über den Seitenabzug bei (c) isolierten PSAs ergab folgende gewichtsbezogene Zusammensetzung:

| 99,97 Gew.-% | PSA |
| 13 ppm | Benzoesäure |
| <10 ppm | Maleinsäureanhydrid |
| <10 ppm | Citraconsäureanhydrid |
| 0,02 Gew.-% | Phthalsäure |

und der Rest zu 100 Gew.-% sonstige Stoffe.
Die Schmelzfarbzahl betrug 5-10 APHA. Die Hitzefarbzahl wurde zu 10-20 APHA gemessen.

Beispiel 3

Zweites erfindungsgemäßes Beispiel

850 g des rohen PSAs gemäß dem vorangehenden Abschnitt B) wurden der Kolonne stündlich kontinuierlich zugeführt (a). In diesem Zeitraum wurden der Kolonne eine Energiemenge von 675 kJ/kg zugeführt. Bei einem Rücklauf von 330 g (b), einem absoluten Druck von 125 mbar am Kolonnenkopf, einer Temperatur von 184°C am Kolonnenkopf und 232°C am Kolonnensumpf wurden in der gleichen Zeit 840 g gereinigtes PSA über den Seitenabzug bei 220°C entnommen, kondensiert und isoliert (c). Das Rücklaufverhältnis in der Kolonne betrug dementsprechend 0,39 und die Ausbeute an derart gereinigtem PSA betrug 99,6 Gew.-%, bezogen auf das der Kolonne zugeführte rohe PSA. Der Kopfabzug über (d) wurde in eine Kühlfalle kondensiert und betrug ca. 3 g; der Sumpfabzug über (e) betrug ca. 7 g und enthielt die Schwersieder und nichtdestillierbare Anteile. Die Analyse des über den Seitenabzug bei (c) isolierten PSAs ergab folgende gewichtsbezogene Zusammensetzung:

| 99,97 Gew.-% | PSA |
| <10 ppm | Benzoesäure |
| <10 ppm | Maleinsäureanhydrid |
| <10 ppm | Citraconsäureanhydrid |
| 0,02 Gew.-% | Phthalsäure |

und der Rest zu 100 Gew.-% sonstige Stoffe.

Die Schmelzfarbzahl betrug 5-10 APHA. Die Hitzefarbzahl wurde zu 10-20 APHA gemessen.

Tabelle

| Gegenüberstellung der Ergebnisse aus den Beispielen 1 bis 3 | | | | | | | |
|---|---|---|---|---|---|---|---|
| Nr. | RLV | E [kJ/kg] | BS [ppm] | FZ [APHA] | HFZ [APHA] | TS | V [%] |
| 1 (Vergleich) | 0,53 | 720 | 15 | 5-10 | 10-20 | 5 | 2,2 |
| 2 (Erfindung) | 0,44 | 690 | 13 | 5-10 | 10-20 | 10 | 1,0 |
| 3 (Erfindung) | 0,39 | 675 | <10 | 5-10 | 10-20 | 13 | 0,4 |

Nr. Nummer des Beispiels
RLV Rücklaufverhältnis:

$$RLV = \frac{R\ddot{u}cklaufmenge/Zeiteinheit}{Zulauf\ zur\ Kolonne/Zeiteinheit} \qquad (1)$$

E Energieeintrag in die Kolonne in kJ/kg rohes PSA
BS Gehalt an Benzoesäure im destillierten PSA [ppm]
FZ Schmelzfarbzahl des destillierten PSAs nach Hazen [APHA]
HFZ Hitzefarbzahl des destillierten PSA nach Hazen [APHA]
TS Zahl der theoretischen Trennstufen oberhalb der Roh-PSA-Zuleitung (a)
V PSA-Verlust bezogen auf die mit dem Roh-PSA der Destillationskolonne zugeführte PSA-Menge

**Patentansprüche**

1. Verfahren zur Herstellung von spezifikationsgerechtem Phthalsäureanhydrid durch die destillative Reinigung von rohem Phthalsäureanhydrid bei vermindertem Druck, wobei man das rohe Phthalsäureanhydrid der Destillationskolonne oberhalb eines Seitenabzugs zuführt, die Leichtsieder am Kopf der Kolonne oder in der Nähe des Kopfes der Kolonne abzieht und das spezifikationsgerechte Phthalsäureanhydrid aus dem Seitenabzug der Kolonne entnimmt, **dadurch gekennzeichnet, dass** man eine Destillationskolonne verwendet, deren Anzahl an oberhalb der Zuleitung des rohen Phthalsäureanhydrids in die Destillationskolonne befindlichen theoretischen Trennstufen 10 bis 20 beträgt und die Kolonne bei einem Rücklaufverhältnis von 0,1 bis 0,5 betreibt.

2. Verfahren gemäß Anspruch 1, **dadurch gekennzeichnet, dass** man die Kolonne bei einem Rücklaufverhältnis von 0,2 bis 0,45 betreibt.

3. Verfahren gemäß Anspruch 1, **dadurch gekennzeichnet, dass** man die Kolonne bei einem Rücklaufverhältnis von 0,25 bis 0,4 betreibt.

**4.** Verfahren nach Anspruch 1, **dadurch gekennzeichnet, dass** man eine Destillationskolonne verwendet, deren Anzahl an oberhalb der Zuleitung des rohen Phthalsäureanhydrids in die Destillationskolonne befindlichen theoretischen Trennstufen 10 bis 15 beträgt.

**5.** Verfahren nach Anspruch 1, **dadurch gekennzeichnet, dass** man das spezifikationsgerechte Phthalsäureanhydrid dem Seitenabzug der Destillationskolonne in gasförmiger Form entnimmt.

**6.** Verfahren nach Anspruch 1, **dadurch gekennzeichnet, dass** man als Destillationskolonne eine mit einem Fallfilmverdampfer betriebene Bodenkolonne verwendet.

**7.** Verfahren nach Anspruch 1, **dadurch gekennzeichnet, dass** man als Destillationskolonne eine Bodenkolonne verwendet, an der am Seitenabzug innerhalb oder außerhalb der Kolonne Tropfenabscheider installiert sind.

**Claims**

**1.** A process for the preparation of on-spec phthalic anhydride by the distillative purification of crude phthalic anhydride at reduced pressure, where the crude phthalic anhydride is passed to the distillation column above a side take-off, the low-boiling components are removed at the top of the column or in the vicinity of the top of the column and the on-spec phthalic anhydride is removed from the side take-off of the column, wherein a distillation column is used whose number of theoretical plates located above the supply of the crude phthalic anhydride into the distillation column is 10 to 20 and the column is operated at a reflux ratio of from 0.1 to 0.5.

**2.** The process according to claim 1, wherein the column is operated at a reflux ratio of from 0.2 to 0.45.

**3.** The process according to claim 1, wherein the column is operated at a reflux ratio of from 0.25 to 0.4.

**4.** The process according to claim 1, wherein a distillation column is used whose number of theoretical plates located above the supply of the crude phthalic anhydride into the distillation column is 10 to 15.

**5.** The process according to claim 1, wherein the on-spec phthalic anhydride is removed from the side take-off of the distillation column in gaseous form.

**6.** The process according to claim 1, wherein the distillation column used is a tray column operated with a falling-film evaporator.

**7.** The process according to claim 1, wherein the distillation column used is a tray column on which drop separators are installed at the side take-off inside or outside of the column.

**Revendications**

**1.** Procédé de fabrication d'anhydride d'acide phtalique conforme aux spécifications par purification par distillation d'anhydride d'acide phtalique brut sous pression réduite, dans lequel on achemine l'anhydride d'acide phtalique brut à la colonne de distillation au-dessus d'un soutirage latéral, on extrait les substances à bas point d'ébullition à la tête de colonne ou à proximité de la tête de colonne et on retire l'anhydride d'acide phtalique conforme aux spécifications par le soutirage latéral de la colonne, **caractérisé en ce que** l'on utilise une colonne de distillation dont le nombre d'étages de séparation théoriques se trouvant au-dessus de l'acheminement de l'anhydride d'acide phtalique brut dans la colonne de distillation est de 10 à 20 et **en ce que** l'on fait fonctionner la colonne avec un rapport de reflux de 0,1 à 0,5.

**2.** Procédé selon la revendication 1, **caractérisé en ce que** l'on fait fonctionner la colonne avec un rapport de reflux de 0,2 à 0,45.

**3.** Procédé selon la revendication 1, **caractérisé en ce que** l'on fait fonctionner la colonne avec un rapport de reflux de 0,25 à 0,4.

**4.** Procédé selon la revendication 1, **caractérisé en ce que** l'on utilise une colonne de distillation dont le nombre d'étages de séparation théoriques au-dessus de l'acheminement de l'anhydride d'acide phtalique brut dans la colonne de distillation est de 10 à 15.

5. Procédé selon la revendication 1, **caractérisé en ce que** l'on retire l'anhydride d'acide phtalique conforme aux spécifications sous forme gazeuse par le soutirage latéral de la colonne de distillation.

6. Procédé selon la revendication 1, **caractérisé en ce que** l'on utilise comme colonne de distillation une colonne à plateaux qui fonctionne avec un évaporateur à film tombant.

7. Procédé selon la revendication 1, **caractérisé en ce que** l'on utilise comme colonne de distillation une colonne à plateaux sur laquelle sont installés des séparateurs de gouttes sur le soutirage latéral à l'intérieur ou à l'extérieur de la colonne.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4547578 A
- US 4008255 A
- WO 0114308 A
- EP 1233012 A

**In der Beschreibung aufgeführte Nicht-Patentliteratur**

- **H. SUTER.** Phthalsäureanhydrid und seine Verwendung. Dr. Dietrich Steinkopff Verlag, 1972
- Ullmann's Encyclopedia of Industrial Chemistry. VCH Verlagsgesellschaft mbH, 1992, vol. A20, 5
- **KIRK-OTHMER.** Encyclopedia of Chemical Technology. John Wiley & Sons, 1996, vol. 18, 997-1006
- **RUHRÖL.** *Europa-Chemie,* 1965, vol. 21, 7
- Organikum. VEB Deutscher Verlag der Wissenschaften, 1975, 42-4450-60
- Grundoperationen chemischer Verfahrenstechnik. **VAUCK ; MÜLLER.** Gegenstromdestillation. Deutscher Verlag für Grundstoffindustrie, 2000, 710-761
- Encyclopedia of Chemical Technology. **KIRK-OTHMER.** Distillation. John Wiley & Sons, 1993, 311-358